# EUROPEAN PATENT APPLICATION

(11) **EP 4 442 267 A1**
(43) Date of publication of application: **09.10.2024**
(21) Application number: 23166323.8
(22) Date of filing: 03.04.2023
(51) Int. Cl.: A61K 36/27, A61K 36/42, A61K 36/48, A61K 36/51, A61K 36/54, A61K 36/61, A61K 36/67, A61K 36/73, A61K 36/736, A61K 36/80, A61K 36/9066, A61P 3/10, A61K 36/47, A61K 36/00

(54) **MEDICINAL PLANT COMPOSITIONS / NUTRACEUTICAL FOR TREATING DIABETES PATIENTS**

(71) Applicant: DiaCure GmbH, 82110 Germering (DE)
(72) Inventor: Hallweg, Friedrich T., 82110 Germering (DE); Kapoor, Rohan, 65185 Wiesbaden (DE)
(74) Representative: Kraus & Lederer PartGmbB

(57) **Abstract**

The present invention relates to a composition comprising a) *Momordica Charantia* extract, b) *Syzygium Cumini* extract, c) *Swertia Chirayita* extract, d) *Trigonella Foenum-Graecum* extract, e) *Gymnema Sylvestre* extract, f) *Phyllanthus Emblica* extract, g) *Neopicrorhiza Scrophulariiflora* extract and/or *Picrorhiza Kurroa* extract, h) *Curcuma longa* extract, i) *Cinnamomum Verum* extract, j) *Piper Nigrum* extract and optionally k) Fruit mixture extract, wherein the fruit mixture comprises apple, pear and plum. Furthermore, the present invention relates to the compositions of the present invention for use in the treatment and/or prevention of diabetes.

## Description

### Field of the invention

The present invention relates to a composition comprising a) *Momordica Charantia* extract, b) *Syzygium Cumini* extract, c) *Swertia Chirayita* extract, d) *Trigonella Foenum-Graecum* extract, e) *Gymnema Sylvestre* extract, f) *Phyllanthus Emblica* extract, g) *Neopicrorhiza Scrophulariiflora* extract and/or *Picrorhiza Kurroa* extract, h) *Curcuma longa* extract, i) *Cinnamomum Verum* extract, j) *Piper Nigrum* extract and optionally k) Fruit mixture extract, wherein the fruit mixture comprises apple, pear and plum. Furthermore, the present invention relates to the compositions of the present invention for use in the treatment and/or prevention of diabetes.

### Technical Background

Diabetes is a chronic, metabolic disease characterized by elevated levels of blood glucose (or blood sugar), which leads over time to serious damage to the heart, blood vessels, eyes, kidneys and nerves. The most common is type 2 diabetes, usually in adults, which occurs when the body becomes resistant to insulin or doesn't make enough insulin. In the past 3 decades the prevalence of type 2 diabetes has risen dramatically in countries of all income levels. Type 1 diabetes, once known as juvenile diabetes or insulin-dependent diabetes, is a chronic condition in which the pancreas produces little or no insulin by itself. For people living with diabetes, access to affordable treatment, including insulin, is critical to their survival. There is a globally agreed target to halt the rise in diabetes and obesity by 2025.

In 2021 537 million adults (20-79 years) are living with diabetes which is 1 out of 10 adults. This number is predicted to rise to 643 million by 2030 and 783 million by 2045. Over 3 in 4 adults with diabetes live in low- and middle-income countries. Diabetes is responsible for 6.7 million deaths in 2021. Diabetes caused at least USD 966 billion dollars in health expenditure which is a 316% increase over the last 15 years. 541 million adults have Impaired Glucose Tolerance (IGT), which places them at high risk of type 2 diabetes.

Diabetes mellitus is a complex metabolic disorder resulting from either insulin insufficiency or insulin dysfunction. Type I diabetes (insulin dependent) is caused due to insulin insufficiency caused by lack of functional beta cells in the Pancreas. Patients suffering from this are therefore totally dependent on exogenous source of insulin while patients suffering from Type II diabetes (insulin independent) are unable to respond to insulin and can be treated with dietary changes, exercise and medication. Type II diabetes is the more common form of diabetes constituting 90% of the diabetic population. Symptoms for both diabetic conditions may include: (i) high levels of sugar in the blood; (ii) unusual thirst; (iii) frequent urination; (iv) extreme hunger and loss of weight; (v) blurred vision; (vi) nausea and vomiting; (vii) extreme weakness and tiredness; (viii) irritability, mood changes and a highly possible tendency for osteoporosis of the jaws with subsequent loss of teeth and advanced periodontitis, etc.

Diabetes is a multifactorial disease leading to several complications, and therefore it mostly demands a multitherapeutic approach. Patients of diabetes either do not produce sufficient volumes of insulin or their cells and metabolism does not respond to insulin. In case of incapability to produce insulin, patients are distributed insulin by injection. In case the metabolism and cells do not respond to insulin, many different medicines and nutraceuticals have been developed, taking into consideration the most probable disturbances in carbohydrate-metabolism.

For example, to manage post-prandial hyper-glycaemia at digestive level, glucosidase inhibitors such as acarbose (a pseudotranssaccharid), miglitol (an anti-diabeticum) and voglibose (an alpha-glucosidase-inhibitor) are applied. These inhibit degradation of carbohydrates thereby reducing the glucose absorption by the cells. To enhance glucose uptake by peripheral cells biguanide such as "METFORMIN" is used. Sulphonylureas, like glibenclamide, are insulinotropic and work as secretagogues in the pancreatic cells.

Although several therapies are in use for treatment, there are certain limitations due to high cost and side effects such as development of hypoglycaemia, weight gain, gastrointestinal disturbances, liver toxicity etc. (Dey, L., Anoja, S.A., and Yuan, C-S.: Alternative therapies for type 2 diabetes. Alternative Med. Rev., 7, 45-58, 2002). Based on recent advances and involvement of oxidative stress in complicating diabetes mellitus, efforts are on to find suitable antidiabetic and antioxidant therapy. Many conventional drugs have been derived from prototypic molecules in medicinal plants.

The review "Indian Herbs and Herbal Drugs Used for the Treatment of Diabetes" of M. Modak et al. J. Clin. Biochem. Nutr., 2007, 40, 163-173 describes several drugs comprising medicinal plants as ingredients such as "DIASLTLIN" which comprises *Cassia Auriculata, Coccinia Indica, Curcuma Longa, Emblica officinalis, Gymnema Sylvestre, Momordica Charantia, Scoparia Dulcis, Syzygium Cumini, Tinospora Cordifolia, Trigonella Foenum-Graecum.*

However, there is still a need for improved, cost-efficient treatment and prevention options for diabetes, ill effects of diabetes and secondary complications of diabetes, wherein medicinal plants are used and no further diabetes medicines are necessary.

### Summary of the invention

It is a problem of the present invention to provide an improved composition based on medicinal plants which can be used for treating and preventing diabetes. The composition of the present invention can be used to control blood sugar levels and hyperglycaemia of diabetes patients, even though unhealthy food and drinks containing large amounts of carbohydrates and fat are consumed by the patient. Moreover, the composition is based on medicinal plant extracts as ingredients and offers a cost-efficient and highly reconcilable approach to treat and/or prevent diabetes, in best case without any further medication. The present invention relates to a composition comprising:
a) *Momordica Charantia* extract;
b) *Syzygium Cumini* extract;
c) *Swertia Chirayita* extract;
d) *Trigonella Foenum-Graecum* extract;
e) *Gymnema Sylvestre* extract;
f) *Phyllanthus Emblica* extract;
g) *Neopicrorhiza Scrophulariiflora* extract *and*/*or Picrorhiza Kurroa* extract;
h) *Curcuma longa* extract;
i) *Cinnamomum Verum* extract;
j) *Piper Nigrum* extract;
   and optionally
k) Fruit mixture extract, wherein the fruit mixture comprises apple, pear and plum.

Finally, the present invention relates to the composition according to the present invention for use in the treatment and/or prevention of diabetes.

Furthermore, the composition of the present invention is based on the recognition that it can be used for improving the health and/or the well-being of a diabetes patient and/or for reducing the effects of diabetes and secondary complications of diabetes. Additionally, the composition of the present invention is a cost-efficient and reconcilable approach for treating diabetes, since the amount of costly commercial diabetes medicines having side effects may be reduced by using the medicinal plant-based compositions having less side effects.

### Brief description of the drawings

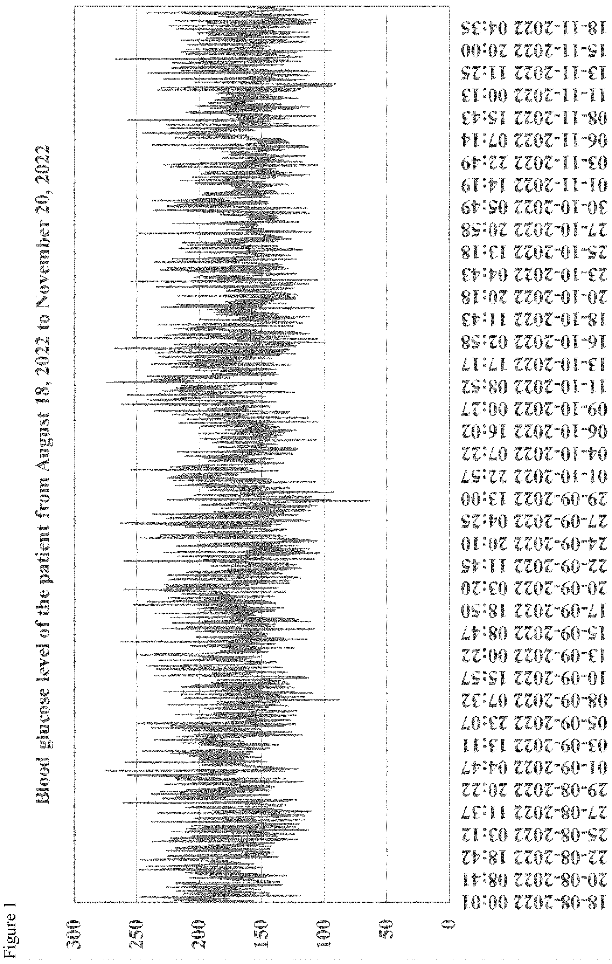

Figure 1 is a diagram of the blood glucose level [mg/dl] of a patient treated with compositions according to the present invention over time in the time period of August 18, 2022 to November 20, 2022.

### Detailed description of the invention

According to the present invention the term "about" means ±10% of the specified numeric value, preferably ±5% and most preferably ±2%.

According to the present invention the term "diabetes" means diabetes mellitus including type 1 diabetes, type 2 diabetes and gestational diabetes.

The present invention relates to a composition comprising:
a) *Momordica Charantia* extract;
b) *Syzygium Cumini* extract;
c) *Swertia Chirayita* extract;
d) *Trigonella Foenum-Graecum* extract;
e) *Gymnema Sylvestre* extract;
f) *Phyllanthus Emblica* extract;
g) *Neopicrorhiza Scrophulariiflora* extract *and*/*or Picrorhiza Kurroa* extract;
h) *Curcuma longa* extract;
i) *Cinnamomum Verum* extract;
j) *Piper Nigrum* extract;
   and optionally
k) Fruit mixture extract, wherein the fruit mixture comprises apple, pear and plum.

According to the present invention a) is Momordica Charantia extract. *Momordica Charantia* is otherwise known as "Karela", "Bitter Melon" or "Balsam Pear". Momordica Charantia extract is commercially available and according to the present invention any commercially available Momordica Charantia extract can be used. The *Momordica Charantia* extract according to the present invention may be produced by extracting *Momordica Charantia* using a hydro-alcohol as a solvent. Preferably, the hydro-alcohol is a mixture of water and ethanol. Subsequently, the hydro-alcohol is removed from the extracted ingredients of *Momordica Charantia.* Preferably, the *Momordica Charantia* extract according to the present invention is a dried solid extract produced by using a hydro-alcohol as an extraction solvent, more preferably a hydro-alcohol which is a mixture of water and ethanol as an extraction solvent.

According to the present invention b) is *Syzygium Cumini* extract. *Syzygium Cumini* is otherwise known as "Eugenia Jambolana", "Aromaticum Java" or "Malabar Plum". *Syzygium Cumini* extract is commercially available and according to the present invention any commercially available *Syzygium Cumini* extract can be used. The *Syzygium Cumini* extract according to the present invention may be produced by extracting *Syzygium Cumini* using a water as a solvent. Subsequently, the water is removed from the extracted ingredients of *Syzygium Cumini.* Preferably, the *Syzygium Cumini* extract according to the present invention is a dried solid extract produced by using water as an extraction solvent.

According to the present invention c) is *Swertia Chirayita* extract. *Swertia Chirayita* is otherwise known as "Kariyatu". *Swertia Chirayita* extract is commercially available and according to the present invention any commercially available *Swertia Chirayita* extract can be used. The *Swertia Chirayita* extract according to the present invention may be produced by extracting *Swertia Chirayita* using a hydro-alcohol as a solvent. Preferably, the hydro-alcohol is a mixture of water and ethanol. Subsequently, the hydro-alcohol is removed from the extracted ingredients of *Swertia Chirayita.* Preferably, the *Swertia Chirayita* extract according to the present invention is a dried solid extract produced by using a hydro-alcohol as an extraction solvent, more preferably a hydro-alcohol which is a mixture of water and ethanol as an extraction solvent.

According to the present invention d) is *Trigonella Foenum-Graecum* extract. *Trigonella Foenum-Graecum* is otherwise known as "Methi" or "Fenugreek". *Trigonella Foenum-Graecum* extract is commercially available and according to the present invention any commercially available *Trigonella Foenum-Graecum extract* can be used. The *Trigonella Foenum-Graecum* extract according to the present invention may be produced by extracting *Trigonella Foenum-Graecum* using a hydro-alcohol as a solvent. Preferably, the hydro-alcohol is a mixture of water and ethanol. Subsequently, the hydro-alcohol is removed from the extracted ingredients of *Trigonella Foenum-Graecum.* Preferably, the *Trigonella Foenum-Graecum* extract according to the present invention is a dried solid extract produced by using a hydro-alcohol as an extraction solvent, more preferably a hydro-alcohol which is a mixture of water and ethanol as an extraction solvent.

According to the present invention e) is *Gymnema Sylvestre* extract. *Gymnema Sylvestre* is otherwise known as "Gudmaar" or "Cowplant". *Gymnema Sylvestre* extract is commercially available and according to the present invention any commercially available *Gymnema Sylvestre* extract can be used. The *Gymnema Sylvestre* extract according to the present invention may be produced by extracting *Gymnema Sylvestre* using a hydro-alcohol as a solvent. Preferably, the hydro-alcohol is a mixture of water and ethanol. Subsequently, the hydro-alcohol is removed from the extracted ingredients of *Gymnema Sylvestre.* Preferably, the *Gymnema Sylvestre* extract according to the present invention is a dried solid extract produced by using a hydro-alcohol as an extraction solvent, more preferably a hydro-alcohol which is a mixture of water and ethanol as an extraction solvent.

According to the present invention f) is *Phyllanthus Emblica* extract. *Phyllanthus Emblica* is otherwise known as "Emblica Officinalis", "Amla", "Myrobalan", "Indian Gooseberry" or "Malacca Tree". *Phyllanthus Emblica* extract is commercially available and according to the present invention any commercially available *Phyllanthus Emblica* extract can be used. The *Phyllanthus Emblica* extract according to the present invention may be produced by extracting *Phyllanthus Emblica* using a water as a solvent.

Subsequently, the water is removed from the extracted ingredients of *Phyllanthus Emblica.* Preferably, the *Phyllanthus Emblica* extract according to the present invention is a dried solid extract produced by using water as an extraction solvent.

According to the present invention g) is *Neopicrorhiza Scrophulariiflora* extract and/or *Picrorhiza Kurroa* extract. The *Neopicrorhiza Scrophulariiflora* according to g) is otherwise known as "Kutki". *Neopicrorhiza Scrophulariiflora* extract is commercially available and according to the present invention any commercially available *Neopicrorhiza Scrophulariiflora* extract can be used. The *Neopicrorhiza Scrophulariiflora* extract according to the present invention may be produced by extracting *Neopicrorhiza Scrophulariiflora* using a hydro-alcohol as a solvent. Preferably, the hydro-alcohol is a mixture of water and ethanol. Subsequently, the hydro-alcohol is removed from the extracted ingredients of *Neopicrorhiza Scrophulariiflora.* Preferably, the *Neopicrorhiza Scrophulariiflora* extract according to the present invention is a dried solid extract produced by using a hydro-alcohol as an extraction solvent, more preferably a hydro-alcohol which is a mixture of water and ethanol as an extraction solvent.

The *Picrorhiza Kurroa* according to g) is otherwise known as *"Kutki*" or *"Picrorhiza Kurroa*". *Picrorhiza Kurroa* extract is commercially available and according to the present invention any commercially available *Picrorhiza Kurroa* extract can be used. The *Picrorhiza Kurroa* extract according to the present invention may be produced by extracting *Picrorhiza Kurroa* using a hydro-alcohol as a solvent. Preferably, the hydro-alcohol is a mixture of water and ethanol. Subsequently, the hydro-alcohol is removed from the extracted ingredients of *Picrorhiza Kurroa.* Preferably, the *Picrorhiza Kurroa* extract according to the present invention is a dried solid extract produced by using a hydro-alcohol as an extraction solvent, more preferably a hydro-alcohol which is a mixture of water and ethanol as an extraction solvent.

According to the present invention h) is *Curcuma longa* extract. *Curcuma longa* otherwise known as "Haldi", "Turmeric", "Curcuma Domestica Valeton". *Curcuma longa* extract is commercially available and according to the present invention any commercially available *Curcuma longa* extract can be used. The *Curcuma longa* extract according to the present invention may be produced by extracting *Curcuma longa* using a hydro-alcohol as a solvent. Preferably, the hydro-alcohol is a mixture of water and ethanol. Subsequently, the hydro-alcohol is removed from the extracted ingredients of *Curcuma longa.* Preferably, the *Curcuma longa* extract according to the present invention is a dried solid extract produced by using a hydro-alcohol as an extraction solvent, more preferably a hydro-alcohol which is a mixture of water and ethanol as an extraction solvent.

According to the present invention i) is *Cinnamomum Verum* extract. *Cinnamomum Verum* otherwise known as "Cinnamon", "Cinnamomum Zeylanicum". *Cinnamomum Verum* extract is commercially available and according to the present invention any commercially available *Cinnamomum Verum* extract can be used. The *Cinnamomum Verum* extract according to the present invention may be produced by extracting *Cinnamomum Verum* using a hydro-alcohol as a solvent. Preferably, the hydro-alcohol is a mixture of water and ethanol. Subsequently, the hydro-alcohol is removed from the extracted ingredients of *Cinnamomum Verum.* Preferably, the *Cinnamomum Verum* extract according to the present invention is a dried solid extract produced by using a hydro-alcohol as an extraction solvent, more preferably a hydro-alcohol which is a mixture of water and ethanol as an extraction solvent.

According to the present invention j) is *Piper Nigrum* extract. *Piper Nigrum* is otherwise known as "Black Pepper". *Piper Nigrum* extract is commercially available and according to the present invention any commercially available *Piper Nigrum* extract can be used. The *Piper Nigrum* extract according to the present invention may be produced by extracting *Piper Nigrum* using a hydro-alcohol as a solvent. Preferably, the hydro-alcohol is a mixture of water and ethanol. Subsequently, the hydro-alcohol is removed from the extracted ingredients of *Piper Nigrum.* Preferably, the *Piper Nigrum* extract according to the present invention is a dried solid extract produced by using a hydro-alcohol as an extraction solvent, more preferably a hydro-alcohol which is a mixture of water and ethanol as an extraction solvent.

According to the present invention k) is a fruit mixture extract, wherein the fruit mixture comprises apple, pear and plum. Most preferably, in the composition according to the present invention as described herein k) is a fruit mixture extract, wherein the fruit mixture consists of apple, pear and plum. The fruit mixture extract, wherein the fruit mixture comprises or consists of apple, pear and plum is of proprietary nature and not commercially available and according to the present invention not any commercially available fruit mixture extract, wherein the fruit mixture comprises or consists of apple, pear and plum can be used. The fruit mixture extract, wherein the fruit mixture comprises or consists of apple, pear and plum, according to the present invention, is produced by extracting a fruit mixture comprising or consisting of apple, pear and plum with CO₂ and mixing it with maltodextrin as a carrier.

Preferably, the composition according to the present invention comprises a) in an amount of from about 0.1wt.% to about 80wt.% of the total weight of the composition, more preferably of from about 0.5wt.% to about 75wt.%, still more preferably of from about 1wt.% to about 70wt.%, even more preferably of from about 2wt.% to about 60wt.%, still even more preferably of from about 5wt.% to about 50wt.%, still even more preferably of from about 7.5wt% to about 45wt.%, still even more preferably of from about 10wt.% to about 40wt.%, still even more preferably of from about 15wt.% to about 35wt.%, still even more preferably of from about 20wt.% to about 35wt.%, and most preferably from about 25wt.% to about 35wt.% of the total weight of the composition.

Preferably, the composition according to the present invention comprises b) in an amount of from about 0.1wt.% to about 80wt.% of the total weight of the composition, more preferably of from about 0.2wt.% to about 75wt.%, still more preferably of from about 0.3wt.% to about 70wt.%, even more preferably of from about 0.4wt.% to about 60wt.%, still even more preferably of from about 0.5wt.% to about 50wt.%, still even more preferably of from about 1wt% to about 45wt.%, still even more preferably of from about 2wt.% to about 40wt.%, still even more preferably of from about 3wt.% to about 35wt.%, still even more preferably of from about 4wt.% to about 30wt.%, still even more preferably of from about 5wt.% to about 20wt.%, still even more preferably of from about 6wt.% to about 15wt.%, most preferably from about 6.5wt.% to about 12wt.% of the total weight of the composition.

Preferably, the composition according to the present invention comprises c) in an amount of from about 0.1wt.% to about 80wt.% of the total weight of the composition, more preferably of from about 0.2wt.% to about 75wt.%, still more preferably of from about 0.3wt.% to about 70wt.%, even more preferably of from about 0.4wt.% to about 60wt.%, still even more preferably of from about 0.5wt.% to about 50wt.%, still even more preferably of from about 1wt% to about 45wt.%, still even more preferably of from about 2wt.% to about 40wt.%, still even more preferably of from about 3wt.% to about 35wt.%, still even more preferably of from about 3.5wt.% to about 30wt.%, still even more preferably of from about 4wt.% to about 20wt.%, still even more preferably of from about 4.5wt.% to about 15wt.%, most preferably from about 5wt.% to about 8.5wt.% of the total weight of the composition.

Preferably, the composition according to the present invention comprises d) in an amount of from about 0.1wt.% to about 80wt.% of the total weight of the composition, more preferably of from about 0.2wt.% to about 75wt.%, still more preferably of from about 0.3wt.% to about 70wt.%, even more preferably of from about 0.4wt.% to about 60wt.%, still even more preferably of from about 0.5wt.% to about 50wt.%, still even more preferably of from about 1wt% to about 45wt.%, still even more preferably of from about 2wt.% to about 40wt.%, still even more preferably of from about 3wt.% to about 35wt.%, still even more preferably of from about 4wt.% to about 30wt.%, still even more preferably of from about 5wt.% to about 20wt.%, still even more preferably of from about 6wt.% to about 15wt.%, most preferably from about 6.5wt.% to about 12wt.% of the total weight of the composition.

Preferably, the composition according to the present invention comprises e) in an amount of from about 0.1wt.% to about 80wt.% of the total weight of the composition, more preferably of from about 0.2wt.% to about 75wt.%, still more preferably of from about 0.3wt.% to about 70wt.%, even more preferably of from about 0.4wt.% to about 60wt.%, still even more preferably of from about 0.5wt.% to about 50wt.%, still even more preferably of from about 1wt% to about 45wt.%, still even more preferably of from about 2wt.% to about 40wt.%, still even more preferably of from about 3wt.% to about 35wt.%, still even more preferably of from about 3.5wt.% to about 30wt.%, still even more preferably of from about 4wt.% to about 20wt.%, still even more preferably of from about 4.5wt.% to about 15wt.%, most preferably from about 5wt.% to about 8.5wt.% of the total weight of the composition.

Preferably, the composition according to the present invention comprises f) in an amount of from about 0.1wt.% to about 80wt.% of the total weight of the composition, more preferably of from about 0.2wt.% to about 75wt.%, still more preferably of from about 0.3wt.% to about 70wt.%, even more preferably of from about 0.4wt.% to about 60wt.%, still even more preferably of from about 0.5wt.% to about 50wt.%, still even more preferably of from about 1wt% to about 45wt.%, still even more preferably of from about 2wt.% to about 40wt.%, still even more preferably of from about 3wt.% to about 35wt.%, still even more preferably of from about 4wt.% to about 30wt.%, still even more preferably of from about 5wt.% to about 20wt.%, still even more preferably of from about 6wt.% to about 15wt.%, most preferably from about 6.5wt.% to about 12wt.% of the total weight of the composition.

Preferably, the composition according to the present invention comprises g) in an amount of from about 0.1wt.% to about 80wt.% of the total weight of the composition, more preferably of from about 0.2wt.% to about 75wt.%, still more preferably of from about 0.3wt.% to about 70wt.%, even more preferably of from about 0.4wt.% to about 60wt.%, still even more preferably of from about 0.5wt.% to about 50wt.%, still even more preferably of from about 1wt% to about 45wt.%, still even more preferably of from about 2wt.% to about 40wt.%, still even more preferably of from about 3wt.% to about 35wt.%, still even more preferably of from about 4wt.% to about 30wt.%, still even more preferably of from about 5wt.% to about 20wt.%, still even more preferably of from about 6wt.% to about 15wt.%, most preferably from about 6.5wt.% to about 12wt.% of the total weight of the composition.

Preferably, the composition according to the present invention comprises h) in an amount of from about 0.1wt.% to about 80wt.% of the total weight of the composition, more preferably of from about 0.2wt.% to about 75wt.%, still more preferably of from about 0.3wt.% to about 70wt.%, even more preferably of from about 0.4wt.% to about 60wt.%, still even more preferably of from about 0.5wt.% to about 50wt.%, still even more preferably of from about 1wt% to about 45wt.%, still even more preferably of from about 2wt.% to about 40wt.%, still even more preferably of from about 3wt.% to about 35wt.%, still even more preferably of from about 4wt.% to about 30wt.%, still even more preferably of from about 5wt.% to about 20wt.%, still even more preferably of from about 6wt.% to about 15wt.%, most preferably from about 6.5wt.% to about 12wt.% of the total weight of the composition.

Preferably, the composition according to the present invention comprises i) in an amount of from about 0.1wt.% to about 80wt.% of the total weight of the composition, more preferably of from about 0.2wt.% to about 75wt.%, still more preferably of from about 0.3wt.% to about 70wt.%, even more preferably of from about 0.4wt.% to about 60wt.%, still even more preferably of from about 0.5wt.% to about 50wt.%, still even more preferably of from about 0.6wt% to about 45wt.%, still even more preferably of from about 0.7wt.% to about 40wt.%, still even more preferably of from about 0.8wt.% to about 35wt.%, still even more preferably of from about 0.9wt.% to about 30wt.%, still even more preferably of from about 1wt.% to about 20wt.%, still even more preferably of from about 1.5wt.% to about 15wt.%, still more preferably about 1.7wt.% to about 10wt.%, still even more preferably 1.8wt.% to about 7.5wt.%, sill even more preferably 2wt.% to about 5wt.%, most preferably from about 2.5wt.% to about 4.5wt.% of the total weight of the composition.

Preferably, the composition according to the present invention comprises j) in an amount of from about 0.1wt.% to about 80wt.% of the total weight of the composition, more preferably of from about 0.2wt.% to about 75wt.%, still more preferably of from about 0.3wt.% to about 70wt.%, even more preferably of from about 0.4wt.% to about 60wt.%, still even more preferably of from about 0.5wt.% to about 50wt.%, still even more preferably of from about 0.5wt% to about 45wt.%, still even more preferably of from about 0.5wt.% to about 40wt.%, still even more preferably of from about 0.5wt.% to about 35wt.%, still even more preferably of from about 0.5wt.% to about 30wt.%, still even more preferably of from about 0.5wt.% to about 20wt.%, still even more preferably of from about 0.5wt.% to about 15wt.%, still more preferably about 0.5wt.% to about 10wt.%, still even more preferably 0.5wt.% to about 7.5wt.%, still even more preferably 0.6wt.% to about 5wt.%, still even more preferably 0.6wt.% to about 2.5wt.%, still even more preferably 0.6wt.% to about 2.0wt.%, still even more preferably 0.6wt.% to about 1.5wt.%, and most preferably from about 0.7wt.% to about 1.0wt.% of the total weight of the composition.

Preferably, the composition according to the present invention comprises a) to j) in the following combinations of amounts as shown in table 1:

**Table 1: Preferred combinations of relative amount of a) to i).**

| | a) [wt.%] | b) [wt.%] | c) [wt.%] | d) [wt.%] | e) [wt.%] | f) [wt.%] | g) [wt.%] | h) [wt.%] | i) [wt.%] | i) [wt.%] |
|---|---|---|---|---|---|---|---|---|---|---|
| 1 | 0.5 to 75 | 0.2 to 75 | 0.2 to 75 | 0.2 to 75 | 0.2 to 75 | 0.2 to 75 | 0.2 to 75 | 0.2 to 75 | 0.2 to 75 | 0.2 to 75 |
| 2 | 1 to 70 | 0.3 to 70 | 0.3 to 70 | 0.3 to 70 | 0.3 to 70 | 0.3 to 70 | 0.3 to 70 | 0.3 to 70 | 0.3 to 70 | 0.3 to 70 |
| 3 | 2 to 60 | 0.4 to 60 | 0.4 to 60 | 0.4 to 60 | 0.4 to 60 | 0.4 to 60 | 0.4 to 60 | 0.4 to 60 | 0.4 to 60 | 0.4 to 60 |
| 4 | 5 to 50 | 0.5 to 50 | 0.5 to 50 | 0.5 to 50 | 0.5 to 50 | 0.5 to 50 | 0.5 to 50 | 0.5 to 50 | 0.5 to 50 | 0.5 to 50 |
| 5 | 5 to 50 | 1 to 45 | 1 to 45 | 1 to 45 | 1 to 45 | 1 to 45 | 1 to 45 | 1 to 45 | 0.6 to 45 | 0.5 to 45 |
| 6 | 7.5 to 45 | 2 to 40 | 2 to 40 | 2 to 40 | 2 to 40 | 2 to 40 | 2 to 40 | 2 to 40 | 0.7 to 40 | 0.5 to 40 |
| 7 | 10 to 40 | 3 to 35 | 3 to 35 | 3 to 35 | 3 to 35 | 3 to 35 | 3 to 35 | 3 to 35 | 0.8 to 35 | 0.5 to 35 |
| 8 | 10 to 40 | 4 to 30 | 3.5 to 30 | 3.5 to 30 | 3.5 to 30 | 4 to 30 | 4 to 30 | 4 to 30 | 0.9 to 30 | 0.5 to 30 |
| 9 | 10 to 40 | 5 to 20 | 4 to 20 | 4 to 30 | 4 to 20 | 5 to 20 | 5 to 20 | 5 to 20 | 1 to 20 | 0.5 to 20 |
| 10 | 10 to 40 | 5 to 20 | 4 to 20 | 5 to 20 | 4 to 20 | 5 to 20 | 5 to 20 | 5 to 20 | 1.5 to 15 | 0.5 to 15 |
| 11 | 10 to 40 | 5 to 20 | 4 to 20 | 5 to 20 | 4 to 20 | 5 to 20 | 5 to 20 | 5 to 20 | 1.7 to 10 | 0.5 to 10 |
| 12 | 10 to 40 | 5 to 20 | 4 to 20 | 5 to 20 | 4 to 20 | 5 to 20 | 5 to 20 | 5 to 20 | 1.8 to 7.5 | 0.5 to 7.5 |
| 13 | 15 to 30 | 6 to 15 | 4.5 to 15 | 6 to 15 | 4.5 to 15 | 6 to 15 | 6 to 15 | 6 to 15 | 2 to 5 | 0.6 to 1.5 |

Preferably, the composition according to the present invention further comprises:
k) Fruit mixture extract, wherein the fruit mixture comprises apple, pear and plum.

Preferably, the composition according to the present invention comprises k) in an amount of from about 0.1wt.% to about 80wt.% of the total weight of the composition, more preferably of from about 0.2wt.% to about 75wt.%, still more preferably of from about 0.3wt.% to about 70wt.%, even more preferably of from about 0.4wt.% to about 60wt.%, still even more preferably of from about 0.5wt.% to about 50wt.%, still even more preferably of from about 0.5wt% to about 45wt.%, still even more preferably of from about 0.5wt.% to about 40wt.%, still even more preferably of from about 0.5wt.% to about 35wt.%, still even more preferably of from about 0.5wt.% to about 30wt.%, still even more preferably of from about 0.5wt.% to about 20wt.%, still even more preferably of from about 0.5wt.% to about 15wt.%, still more preferably about 0.5wt.% to about 10wt.%, still even more preferably 0.5wt.% to about 7.5wt.%, still even more preferably 0.6wt.% to about 6wt.%, and most preferably from about 0.7wt.% to about 5wt.% of the total weight of the composition.

Preferably, the composition according the present invention comprises a) in a total amount of from about 20 mg to about 450 mg, more preferably in a total amount of from about 40 mg to about 400 mg, even more preferably in a total amount of from about 50 mg to about 350 mg, still more preferably in a total amount of from about 70 mg to about 300 mg, even still more preferably in a total amount of from about 100 mg to about 250 mg, even still more preferably in a total amount of from about 120 mg to about 200 mg, even still more preferably in a total amount of from about 140 mg to about 180 mg, most preferably the composition according to the present invention comprises a) in a total amount of 150 mg.

Preferably, the composition according the present invention comprises b) in a total amount of from about 1 mg to about 150 mg, more preferably in a total amount of from about 5 mg to about 125 mg, even more preferably in a total amount of from about 10 mg to about 100 mg, still more preferably in a total amount of from about 20 mg to about 90 mg, even still more preferably in a total amount of from about 30 mg to about 80 mg, even still more preferably in a total amount of from about 40 mg to about 70 mg, even still more preferably in a total amount of from about 45 mg to about 60 mg, most preferably the composition according to the present invention comprises b) in a total amount of 50 mg.

Preferably, the composition according the present invention comprises c) in a total amount of from about 1 mg to about 150 mg, more preferably in a total amount of from about 5 mg to about 125 mg, even more preferably in a total amount of from about 10 mg to about 100 mg, still more preferably in a total amount of from about 20 mg to about 90 mg, even still more preferably in a total amount of from about 25 mg to about 80 mg, even still more preferably in a total amount of from about 30 mg to about 70 mg, even still more preferably in a total amount of from about 35 mg to about 60 mg, most preferably the composition according to the present invention comprises c) in a total amount of 40 mg.

Preferably, the composition according the present invention comprises d) in a total amount of from about 1 mg to about 150 mg, more preferably in a total amount of from about 5 mg to about 125 mg, even more preferably in a total amount of from about 10 mg to about 100 mg, still more preferably in a total amount of from about 20 mg to about 90 mg, even still more preferably in a total amount of from about 30 mg to about 80 mg, even still more preferably in a total amount of from about 40 mg to about 70 mg, even still more preferably in a total amount of from about 45 mg to about 60 mg, most preferably the composition according to the present invention comprises d) in a total amount of 50 mg.

Preferably, the composition according the present invention comprises e) in a total amount of from about 1 mg to about 150 mg, more preferably in a total amount of from about 5 mg to about 125 mg, even more preferably in a total amount of from about 10 mg to about 100 mg, still more preferably in a total amount of from about 20 mg to about 90 mg, even still more preferably in a total amount of from about 25 mg to about 80 mg, even still more preferably in a total amount of from about 30 mg to about 70 mg, even still more preferably in a total amount of from about 35 mg to about 60 mg, most preferably the composition according to the present invention comprises e) in a total amount of 40 mg.

Preferably, the composition according the present invention comprises f) in a total amount of from about 1 mg to about 150 mg, more preferably in a total amount of from about 5 mg to about 125 mg, even more preferably in a total amount of from about 10 mg to about 100 mg, still more preferably in a total amount of from about 20 mg to about 90 mg, even still more preferably in a total amount of from about 30 mg to about 80 mg, even still more preferably in a total amount of from about 40 mg to about 70 mg, even still more preferably in a total amount of from about 45 mg to about 60 mg, most preferably the composition according to the present invention comprises f) in a total amount of 50 mg.

Preferably, the composition according the present invention comprises g) in a total amount of from about 1 mg to about 150 mg, more preferably in a total amount of from about 5 mg to about 125 mg, even more preferably in a total amount of from about 10 mg to about 100 mg, still more preferably in a total amount of from about 20 mg to about 90 mg, even still more preferably in a total amount of from about 30 mg to about 80 mg, even still more preferably in a total amount of from about 40 mg to about 70 mg, even still more preferably in a total amount of from about 45 mg to about 60 mg, most preferably the composition according to the present invention comprises g) in a total amount of 50 mg.

Preferably, the composition according the present invention comprises h) in a total amount of from about 1 mg to about 150 mg, more preferably in a total amount of from about 5 mg to about 125 mg, even more preferably in a total amount of from about 10 mg to about 100 mg, still more preferably in a total amount of from about 20 mg to about 90 mg, even still more preferably in a total amount of from about 30 mg to about 80 mg, even still more preferably in a total amount of from about 40 mg to about 70 mg, even still more preferably in a total amount of from about 45 mg to about 60 mg, most preferably the composition according to the present invention comprises h) in a total amount of 50 mg.

Preferably, the composition according the present invention comprises i) in a total amount of from about 0.1 mg to about 100 mg, more preferably in a total amount of from about 0.5 mg to about 90 mg, even more preferably in a total amount of from about 1 mg to about 80 mg, still more preferably in a total amount of from about 2 mg to about 70 mg, even still more preferably in a total amount of from about 3 mg to about 60 mg, even still more preferably in a total amount of from about 4 mg to about 50 mg, even still more preferably in a total amount of from about 5 mg to about 40 mg, even still more preferably in a total amount of from about 10 mg to about 30 mg, most preferably the composition according to the present invention comprises i) in a total amount of 20 mg.

Preferably, the composition according the present invention comprises j) in a total amount of from about 0.1 mg to about 100 mg, more preferably in a total amount of from about 0.2 mg to about 90 mg, even more preferably in a total amount of from about 0.3 mg to about 80 mg, still more preferably in a total amount of from about 0.4 mg to about 70 mg, even still more preferably in a total amount of from about 0.5 mg to about 60 mg, even still more preferably in a total amount of from about 0.6 mg to about 50 mg, even still more preferably in a total amount of from about 0.7 mg to about 40 mg, even still more preferably in a total amount of from about 0.8 mg to about 30 mg, even still more preferably in a total amount of from about 0.9 mg to about 20 mg, even still more preferably in a total amount of from about 1.0 mg to about 10 mg, even still more preferably in a total amount of from about 1.5 mg to about 7.5 mg, and most preferably the composition according to the present invention comprises j) in a total amount of 5 mg.

Preferably, the composition according to the present invention comprises a) to j) in the following combinations of total amounts as shown in table 2:

**Table 2: Preferred combinations of absolute amounts of a to j).**

| | a) [mg] | b) [mg] | c) [mg] | d) [mg] | e) [mg] | f) [mg] | g) [mg] | h) [mg] | i) [mg] | j) [mg] |
|---|---|---|---|---|---|---|---|---|---|---|
| 1 | 20 to 450 | 1 to 150 | 1 to 150 | 1 to 150 | 1 to 150 | 1 to 150 | 1 to 150 | 1 to 150 | 0.1 to 100 | 0.1 to 100 |
| 2 | 40 to 400 | 5 to 125 | 5 to 125 | 5 to 125 | 5 to 125 | 5 to 125 | 5 to 125 | 5 to 125 | 0.5 to 90 | 0.2 to 90 |
| 3 | 50 to 350 | 10 to 100 | 10 to 100 | 10 to 100 | 10 to 100 | 10 to 100 | 10 to 100 | 10 to 100 | 1 to 80 | 0.3 to 80 |
| 4 | 70 to 300 | 20 to 90 | 20 to 90 | 20 to 90 | 20 to 90 | 20 to 90 | 20 to 90 | 20 to 90 | 2 to 70 | 0.4 to 70 |
| 5 | 150 | 50 | 40 | 50 | 40 | 50 | 50 | 50 | 20 | 5 |

Preferably, the composition according to the present invention comprises k) and the composition according the present invention comprises k) in a total amount of from about 0.1 mg to about 100 mg, more preferably in a total amount of from about 0.2 mg to about 90 mg, even more preferably in a total amount of from about 0.3 mg to about 80 mg, still more preferably in a total amount of from about 0.4 mg to about 70 mg, even still more preferably in a total amount of from about 0.5 mg to about 60 mg, even still more preferably in a total amount of from about 0.6 mg to about 50 mg, even still more preferably in a total amount of from about 0.7 mg to about 40 mg, even still more preferably in a total amount of from about 0.8 mg to about 35 mg, even still more preferably in a total amount of from about 0.9 mg to about 30 mg, even still more preferably in a total amount of from about 1.0 mg to about 25 mg, even still more preferably in a total amount of from about 10 mg to about 25 mg, even still more preferably in a total amount of from about 15 mg to about 20 mg, and most preferably the composition according to the present invention comprises k) in a total amount of 20 mg.

Preferably, the composition according to the present invention comprises a) to k) in the following combinations of total amounts as shown in table 3:

**Table 3: Preferred combinations of absolute amounts of a to k).**

| | a) [mg] | b) [mg] | c) [mg] | d) [mg] | e) [mg] | f) [mg] | g) [mg] | h) [mg] | i) [mg] | i) [mg] | k) [mg] |
|---|---|---|---|---|---|---|---|---|---|---|---|
| 1 | 20 to 450 | 1 to 150 | 1 to 150 | 1 to 150 | 1 to 150 | 1 to 150 | 1 to 150 | 1 to 150 | 0.1 to 100 | 0.1 to 100 | 0.1 to 100 |
| 2 | 40 to 400 | 5 to 125 | 5 to 125 | 5 to 125 | 5 to 125 | 5 to 125 | 5 to 125 | 5 to 125 | 0.5 to 90 | 0.2 to 90 | 0.2 to 90 |
| 3 | 50 to 350 | 10 to 100 | 10 to 100 | 10 to 100 | 10 to 100 | 10 to 100 | 10 to 100 | 10 to 100 | 1 to 80 | 0.3 to 80 | 0.3 to 80 |
| 4 | 70 to 300 | 20 to 90 | 20 to 90 | 20 to 90 | 20 to 90 | 20 to 90 | 20 to 90 | 20 to 90 | 2 to 70 | 0.4 to 70 | 0.4 to 70 |
| 5 | 150 | 50 | 40 | 50 | 40 | 50 | 50 | 50 | 20 | 5 | 20 |

Preferably, the composition according to the present invention is in an oral dosage form, more preferably in a solid oral dosage form, more preferably in a solid oral dosage form selected from the group consisting of: a powder, a tablet and a capsule; most preferably the composition is in a capsule form. According to the present invention the capsule may be any capsule known in the art. Preferably, the capsule is a gel-capsule or a veggie-capsule.

For example, capsules according to the present invention are based on plant-based cellulose HPMC (Hydroxypropylmetylcellulose) or Pullulan.

According to the present invention the capsules may have the following size:
Size = Length 23mm Diameter 7mm
Size = Length 21mm Diameter 6mm

### GEL CAPSULES:

According to the present invention gel capsules may be based on the OptiShell^{®} Technology or the RP Scherer Softgel technology by the company Catalent.

In a preferred embodiment, the composition according to the present invention as described herein consists of:
a) *Momordica Charantia* extract;
b) *Syzygium Cumini* extract;
c) *Swertia Chirayita* extract;
d) *Trigonella Foenum-Graecum extract;*
e) *Gymnema Sylvestre* extract;
f) *Phyllanthus Emblica* extract;
g) *Neopicrorhiza Scrophulariiflora* extract and/or *Picrorhiza Kurroa* extract;
h) *Curcuma longa* extract;
i) *Cinnamomum Verum* extract; and
j) *Piper Nigrum* extract.

In a more preferred embodiment, the composition according to the present invention as described herein consists of:
a) *Momordica Charantia* extract;
b) *Syzygium Cumini* extract;
c) *Swertia Chirayita* extract;
d) *Trigonella Foenum-Graecum extract;*
e) *Gymnema Sylvestre* extract;
f) *Phyllanthus Emblica* extract;
g) *Neopicrorhiza Scrophulariiflora* extract;
h) *Curcuma longa* extract;
i) *Cinnamomum Verum* extract; and
j) *Piper Nigrum* extract.

In a more preferred embodiment, the composition according to the present invention as described herein consists of:
a) *Momordica Charantia* extract;
b) *Syzygium Cumini* extract;
c) *Swertia Chirayita* extract;
d) *Trigonella Foenum-Graecum extract;*
e) *Gymnema Sylvestre* extract;
f) *Phyllanthus Emblica* extract;
g) *Picrorhiza Kurroa* extract;
h) *Curcuma longa* extract;
i) *Cinnamomum Verum* extract; and
j) *Piper Nigrum* extract.

In a still more preferred embodiment, the composition according to the present invention as described herein consists of:
a) *Momordica Charantia* extract;
b) *Syzygium Cumini* extract;
c) *Swertia Chirayita* extract;
d) *Trigonella Foenum-Graecum* extract;
e) *Gymnema Sylvestre* extract;
f) *Phyllanthus Emblica* extract;
g) *Neopicrorhiza Scrophulariiflora* extract *and*/*or Picrorhiza Kurroa* extract;
h) *Curcuma longa* extract;
i) *Cinnamomum Verum* extract;
j) *Piper Nigrum* extract;
   and
k) Fruit mixture extract, wherein the fruit mixture comprises apple, pear and plum.

In a still more preferred embodiment, the composition according to the present invention as described herein consists of:
a) *Momordica Charantia* extract;
b) *Syzygium Cumini* extract;
c) *Swertia Chirayita* extract;
d) *Trigonella Foenum-Graecum* extract;
e) *Gymnema Sylvestre* extract;
f) *Phyllanthus Emblica* extract;
g) *Neopicrorhiza Scrophulariiflora* extract;
h) *Curcuma longa* extract;
i) *Cinnamomum Verum* extract;
j) *Piper Nigrum* extract;
   and
k) Fruit mixture extract, wherein the fruit mixture comprises apple, pear and plum.

In the most preferred embodiment, the composition according to the present invention as described herein consists of:
a) *Momordica Charantia* extract;
b) *Syzygium Cumini* extract;
c) *Swertia Chirayita* extract;
d) *Trigonella Foenum-Graecum* extract;
e) *Gymnema Sylvestre* extract;
f) *Phyllanthus Emblica* extract;
g) *Picrorhiza Kurroa* extract;
h) *Curcuma longa* extract;
i) *Cinnamomum Verum* extract;
j) *Piper Nigrum* extract;
   and
k) Fruit mixture extract, wherein the fruit mixture comprises apple, pear and plum.

Preferably, the composition according to the present invention as defined herein comprises the ingredients a) to j) in an amount of about 400 mg up to about 750 mg. More preferably, the composition comprises a) to j) in an amount of about 505 mg.

Preferably, the composition of the present invention as described herein is in a capsule as a solid oral dosage form and contains a) to j) in an amount of about 400mg up to about 750mg. More preferably, the composition of the present invention as described herein is in a capsule as a solid oral dosage form and comprises a) to j) in an amount of about 505 mg.

Preferably, the composition according to the present invention as defined herein comprises the ingredients a) to k) in an amount of about 400 mg up to about 750 mg. More preferably, the composition comprises a) to k) in an amount of about 525 mg.

Preferably, the composition of the present invention as described herein is in a capsule as a solid oral dosage form and contains a) to k) in an amount of about 400mg up to about 750mg. More preferably, the composition of the present invention as described herein is in a capsule as a solid oral dosage form and comprises a) to k in an amount of about 525 mg.

In some embodiments, the composition of the present invention as described herein may further comprise:
m) one or more pharmaceutically acceptable excipient(s).

The one or more pharmaceutically acceptable excipient(s) may be any pharmaceutically acceptable excipient(s) known in the art.

In case the composition according to the present invention is in form of a tablet or capsule, the tablet or capsule preferably has a weight of from about 100mg to about 2000mg, more preferably of from about 200mg to about 1500mg, still more preferably of from about 300mg to about 1000mg, even more preferably of from about 400mg to about 1000mg and most preferably of from about 500mg to about 1000mg.

Furthermore, the present invention relates to the compositions as defined herein for use in the treatment and/or prevention of diabetes.

Specifically, the present invention relates to a composition comprising:
a) *Momordica Charantia* extract;
b) *Syzygium Cumini* extract;
c) *Swertia Chirayita* extract;
d) *Trigonella Foenum-Graecum* extract;
e) *Gymnema Sylvestre* extract;
f) *Phyllanthus Emblica* extract;
g) *Neopicrorhiza Scrophulariiflora* extract *and*/*or Picrorhiza Kurroa* extract;
h) *Curcuma longa* extract;
i) *Cinnamomum Verum* extract;
j) *Piper Nigrum* extract;
   and optionally
k) Fruit mixture extract, wherein the fruit mixture comprises apple, pear and plum. for use in the treatment and/or prevention of diabetes.

In a preferred embodiment, the composition for use in the treatment and/or prevention of diabetes according to the present invention consists of:
a) *Momordica Charantia* extract;
b) *Syzygium Cumini* extract;
c) *Swertia Chirayita* extract;
d) *Trigonella Foenum-Graecum* extract;
e) *Gymnema Sylvestre* extract;
f) *Phyllanthus Emblica* extract;
g) *Neopicrorhiza Scrophulariiflora* extract *and*/*or Picrorhiza Kurroa* extract;
h) *Curcuma longa* extract;
i) *Cinnamomum Verum* extract;
j) *Piper Nigrum* extract;
   and
k) Fruit mixture extract, wherein the fruit mixture comprises apple, pear and plum.

In the most preferred embodiment, the composition for use in the treatment and/or prevention of diabetes according to the present invention consists of:
a) *Momordica Charantia* extract;
b) *Syzygium Cumini* extract;
c) *Swertia Chirayita* extract;
d) *Trigonella Foenum-Graecum* extract;
e) *Gymnema Sylvestre* extract;
f) *Phyllanthus Emblica* extract;
g) *Picrorhiza Kurroa* extract;
h) *Curcuma longa* extract;
i) *Cinnamomum Verum* extract;
j) *Piper Nigrum* extract;
   and
k) Fruit mixture extract, wherein the fruit mixture comprises apple, pear and plum.

Preferably, the composition for use in the treatment and/or prevention of diabetes according to the present invention is for use in the treatment and/or prevention of type 2 diabetes.

Preferably, the composition for use in the treatment and/or prevention of diabetes according to the present invention is in an oral dosage form, more preferably in a solid oral dosage form, more preferably in a solid oral dosage form selected from the group consisting of: a powder, a tablet and a capsule; most preferably the composition is in a capsule form.

Preferably, the composition according to the present invention for use in the treatment and/or prevention of diabetes is in a tablet or capsule dosage form comprising the ingredients a) to j) in an amount of about 505mg and is used according to the following dosage regimen shown in table 3 depending on the blood glucose level of the patient:

Preferably, the composition according to the present invention for use in the treatment and/or prevention of diabetes is in a tablet or capsule dosage form comprising the ingredients a) to k) in an amount of about 525mg and is used according to the following dosage regimen shown in table 4 depending on the blood glucose level of the patient:

**Table 4:**

| Blood Glucose | Capsules or tablets / Day | | | When? | |
|---|---|---|---|---|---|
| | | | | | |
| mg/dl | | | 30 Minutes after meal | | |
| ≤ 120 | | 1-2 | Breakfast | Lunch | Supper |
| | | | 1 | / | 1 |
| 120-220 | | 2-4 | Breakfast | Lunch | Supper |
| | | | 1 | 1 | 1 |
| ≥ 220 | | 6 | Breakfast | Lunch | Supper |
| | | | 2 | 2 | 2 |

### Examples

### Example 1: Formulation Examples

The following specific compositions are formulation examples for powders and capsules.

For the following formulation examples commercially available compounds a) to l) have been used from the following suppliers:
a) *Momordica Charantia* extract from the supplier NaturMed Scientific GmbH and/or VIDYA EUROPE S.A. was used.
b) *Syzygium Cumini* extract from the supplier NaturMed Scientific GmbH and/or VIDYA EUROPE S.A.
c) *Swertia Chirayita* extract from the supplier NaturMed Scientific GmbH and/or VIDYA EUROPE S.A.
d) *Trigonella Foenum-Graecum extract* from the supplier NaturMed Scientific GmbH and/or VIDYA EUROPE S.A.
e) *Gymnema Sylvestre* extract from the supplier NaturMed Scientific GmbH and/or VIDYA EUROPE S.A.
f) *Phyllanthus Emblica* extract from the supplier NaturMed Scientific GmbH and/or VIDYA EUROPE S.A.
g) *Neopicrorhiza Scrophulariiflora* extract and *Picrorhiza Kurroa* extract from the supplier NaturMed Scientific GmbH and/or VIDYA EUROPE S.A.
h) *Curcuma longa* extract from the supplier NaturMed Scientific GmbH and/or VIDYA EUROPE S.A.
i) *Cinnamomum Verum* extract from the supplier NaturMed Scientific GmbH and/or VIDYA EUROPE S.A.
j) *Piper Nigrum-extract* from the supplier NaturMed Scientific GmbH was used;
k) Fruit mixture extract, wherein the fruit mixture consists of apple, pear and plum from the supplier NaturMed Scientific GmbH was used.

For providing powders according to the formulation examples 1 to 4 the solid ingredients a) to m) are mixed together in the respective amounts. For providing the formulation examples 1 to 4 in capsule form, the respective powders are filled in a gel-capsule, the gel-capsule is a capsule of the OptiShell^{®} Technology or the RP Scherer Softgel technology by the company Catalent. The formulation examples 1 to 4 are shown in tables 5 to 8 below.

**Table 4:**

| Formulation Example 1 produced in powder form as well as in a gel-capsule. | | |
|---|---|---|
| Ingredient | Name of ingredient | Amount[mg] |
| a) | *Momordica Charantia* extract | 150.000 |
| b) | *Syzygium Cumini* extract | 50.000 |
| c) | *Swertia Chirayita* extract | 40.000 |
| d) | *Trigonella Foenum-Graecum* extract | 50.000 |
| e) | *Gymnema Sylvestre* extract | 40.000 |
| f) | *Phyllanthus Emblica* extract | 50.000 |
| g) | *Picrorhiza Kurroa* extract | 50.000 |
| h) | *Curcuma Longa* extract | 50.000 |
| i) | *Cinnamomum Verum* extract | 20.000 |
| j) | *Piper Nigrum* extract | 5.000 |
| | **Total** | **505.000** |

**Table 5:**

| Formulation Example 2 produced in powder form as well as in a gel-capsule. | | |
|---|---|---|
| Ingredient | Name of ingredient | Amount [mg] |
| a) | *Momordica Charantia* extract | 150.000 |
| b) | *Syzygium Cumini* extract | 50.000 |
| c) | *Swertia Chirayita* extract | 40.000 |
| d) | *Trigonella Foenum-Graecum* extract | 50.000 |
| e) | *Gymnema Sylvestre* extract | 40.000 |
| f) | *Phyllanthus Emblica* extract | 50.000 |
| g) | *Neopicrorhiza Scrophulariiflora* extract | 50.000 |
| h) | *Curcuma Longa* extract | 50.000 |
| i) | *Cinnamomum Verum* extract | 20.000 |
| j) | *Piper Nigrum* extract | 5.000 |
| | **Total** | **505.000** |

**Table 6:**

| Formulation Example 3 produced in powder form as well as in a gel-capsule. | | |
|---|---|---|
| Ingredient | Name of ingredient | Amount [mg] |
| a) | *Momordica Charantia* extract | 150.000 |
| b) | *Syzygium Cumini* extract | 50.000 |
| c) | *Swertia Chirayita* extract | 40.000 |
| d) | *Trigonella Foenum-Graecum* extract | 50.000 |
| e) | *Gymnema Sylvestre* extract | 40.000 |
| f) | *Phyllanthus Emblica* extract | 50.000 |
| g) | *Neopicrorhiza Scrophulariiflora* extract | 50.000 |
| h) | *Curcuma Longa* extract | 50.000 |
| i) | *Cinnamomum Verum* extract | 20.000 |
| j) | *Piper Nigrum* extract | 5.000 |
| k) | Fruit mixture extract of apple, pear and plum | 20.000 |
| | **Total** | **525.000** |

**Table 7:**

| Formulation Example 4 produced in powder form as well as in a gel-capsule. | | |
|---|---|---|
| Ingredient | Name of ingredient | Amount [mg] |
| a) | *Momordica Charantia* extract | 150.000 |
| b) | *Syzygium Cumini* extract | 50.000 |
| c) | *Swertia Chirayita* extract | 40.000 |
| d) | *Trigonella Foenum-Graecum* extract | 50.000 |
| e) | *Gymnema Sylvestre* extract | 40.000 |
| f) | *Phyllanthus Emblica* extract | 50.000 |
| g) | *Neopicrorhiza Scrophulariiflora* extract | 50.000 |
| h) | *Curcuma Longa* extract | 50.000 |
| i) | *Cinnamomum Verum* extract | 20.000 |
| j) | *Piper Nigrum* extract | 5.000 |
| k) | Fruit mixture extract of apple, pear and plum | 20.000 |
| | **Total** | **525.000** |

### Example 2: Treatment of a diabetes patient

### Methods:

The blood glucose levels of the patient before the treatment phase and during the 18 months treatment phase were initially monitored each day in the morning (breakfast time), at lunch time, in the evening (dinner time), and at night before going to bed. In the final phase of the treatment, the patient was submitted to live-surveillance of blood glucose via the Abbot Freestyle Libre 3^{®} - System.

### Treatment:

A patient diagnosed with type 2 diabetes was treated with the compositions according to formulation examples 1 to 4 as shown above.

For several years before the beginning of the treatment with the composition according to the present invention the patient was on diet, trying to avoid food and drinks containing fats and carbohydrates and was already taking two commercially available medicines:
METFORMIN 500mg twice daily and TRULICITY 0.75mg once a week, because the treatment with only METFORMIN in combination with the diet was not sufficient to control the blood glucose level of the patient.

Before the treatment during the diet, the patient was administered METFORMIN 500mg twice daily and 0.75mg TRULICITY once a week and in combination with the diet the patient was able to control the blood glucose level, but suffered some side effects from the commercial medicines such as headache. The general well-being of the patient suffered due to the strict diet and the side effects of the commercially available medicines.

At the beginning of the treatment phase the patient abandoned the strict diet and consumed foods and drinks containing fat and carbohydrates such as ice cream, chocolate, gummy bears, fruits, bread, ice coffee and fruit juices. However, the patient was still able to control the blood glucose level in a healthy manner by taking the compositions according to the present invention orally in form of 2 capsules per serving, 3 to 4 times a day, after each meal or snack.

Soon after the beginning of the treatment, the treatment showed excellent results and the patient was able to control the blood glucose level despite the fact that the strict diet was abandoned. However, the patient still suffered from the side effects of the commercially available medicines. Therefore, the patient replaced the TRULICITY 0.75mg once a week with OZEMPIC 0.5mg once a week and reduced the dosage of METFORMIN 500mg twice a day to METFORMIN 500mg once a day. After the reduction of the dosage of the commercially available medicines and the abandonment of the strict diet the patient was still able to control the blood glucose level in a healthy manner by taking the compositions according to according to the present invention orally in form of 2 capsules per serving, 3 to 4 times a day, after each meal or snack. Furthermore, after the reduction of the dosage of the commercially available medicines the patient did not suffer from any side effects.

During the treatment phase of the patient for about 18 months the patient was treated separately with a composition according to formulation example 1, with the composition according to formulation example 2, with the composition according to formulation example 3 and with the composition according formulation example 4. Only one composition according to the present invention and according to the formulation examples 1 to 4 was administered at a time for at least several weeks. The patient was not treated with different compositions according to the present invention at the same time. All of the compositions according to formulation examples 1 to 4 in combination with OZEMPIC 0.5mg once a week and METFORMIN 500mg once a day showed excellent results in controlling the blood glucose level of the patient. Moreover, it was possible for the patient to control the blood glucose level in a healthy manner having no side effects from the reduced dosage of the commercial medicines by using one of the compositions according to formulation examples 1 to 4.

For comparison, the blood glucose levels before the starting the one-year diet and during the one-year diet are shown in the following table 8:

**Table 8:**

| | Date | Daytime | Blood glucose level [mg/dl] |
|---|---|---|---|
| At the day of diabetes diagnosis (before diet) | 26/09/2017 | Morning | 278 |
| At the last day of medication only. | 14/05/2021 | Morning | 319 |
| At the beginning of the treatment. | 14/05/2021 | Morning | 319 |
| At the end of the test treatment. | 20/11/2022 | Morning | 146 |
| Lowest Blood glucose level in test: | 04/20/2022 | Early Morning | 53 |

During the entire treatment phase in which the patient was treated with the compositions of the present invention the blood glucose levels were measured and the patient was able to control the blood glucose level in healthy range. As an example, for the control of the blood glucose level in a healthy range, Figure 1 shows the blood glucose levels measured by live-surveillance of blood glucose via the Abbot Freestyle Libre 3^{®} - System every 5 minutes, for each day, of from August 18, 2022 to November 20, 2022.

As can be seen from the data of figure 1, in the time period of over 3 months the blood glucose level was controlled in a healthy range by taking the composition according to the present invention orally in form of 2 capsules per serving, 3 to 4 times a day, after each meal or snack, even so the patient did not follow a strict diet and the dosage of the commercially available medicines was reduced as stated above. Moreover, after reducing the METFORMIN dosage and taking the composition of the present invention the side effects which the patient experienced disappeared. This demonstrates that the composition according to the present invention is a cost-efficient and reconcilable approach, since the dosage of the expensive commercial diabetes medicines having side effects can be lowered, even if the patient does not follow a strict diet. The reduced side effects and the possibility to abandon the strict diet led to a significant increase in quality of life and general well-being of the patient.

Currently, the patient discontinued the treatment with MEFORMIN 500mg completely, thus resulting in slightly increased, but still healthy blood glucose values.

Clinical studies using the composition according to the present invention for use in the treatment of diabetes patients will be conducted in the future.

## Claims

1. A composition comprising:
a) *Momordica Charantia* extract;
b) *Syzygium Cumini* extract;
c) *Swertia Chirayita* extract;
d) *Trigonella Foenum-Graecum* extract;
e) *Gymnema Sylvestre* extract;
f) *Phyllanthus Emblica* extract;
g) *Neopicrorhiza Scrophulariiflora* extract *and*/*or Picrorhiza Kurroa* extract;
h) *Curcuma longa* extract;
i) *Cinnamomum Verum* extract;
j) *Piper Nigrum* extract;
and optionally
k) Fruit mixture extract, wherein the fruit mixture comprises apple, pear and plum.

2. The composition according to claim 1, wherein the composition comprises a) in an amount of from about 0.1wt.% to about 80wt.% of the total weight of the composition;
wherein the composition comprises b) in an amount of from about 0.1wt.% to about 80wt.% of the total weight of the composition;
wherein the composition comprises c) in an amount of from about 0.1wt.% to about 80wt.% of the total weight of the composition;
wherein the composition comprises e) in an amount of from about 0.1wt.% to about 80wt.% of the total weight of the composition;
wherein the composition comprises f) in an amount of from about 0.1wt.% to about 80wt.% of the total weight of the composition;
wherein the composition comprises g) in an amount of from about 0.1wt.% to about 80wt.% of the total weight of the composition;
wherein the composition comprises h) in an amount of from about 0.1wt.% to about 80wt.% of the total weight of the composition;
wherein the composition comprises i) in an amount of from about 0.1wt.% to about 80wt.% of the total weight of the composition; and
wherein the composition comprises j) in an amount of from about 0.1wt.% to about 80wt.% of the total weight of the composition.

3. The composition according to claim 1 or 2, wherein the composition comprises a) in an amount of from about 25wt.% to about 35wt.% of the total weight of the composition.

4. The composition according to anyone of claims 1 to 3, wherein the composition comprises b) in an amount of from about 6.5wt.% to about 12wt.% of the total weight of the composition.

5. The composition according to anyone of claims 1 to 4, wherein the composition comprises c) in an amount of from about 5wt.% to about 8.5wt.% of the total weight of the composition.

6. The composition according to anyone of claims 1 to 5, wherein the composition comprises d) in an amount of from about 6.5wt.% to about 12wt.% of the total weight of the composition.

7. The composition according to anyone of claims 1 to 6, wherein the composition comprises e) in an amount of from about 5wt.% to about 8.5wt.% of the total weight of the composition.

8. The composition according to anyone of claims 1 to 7, wherein the composition comprises f) in an amount of from about 6.5wt.% to about 12wt.% of the total weight of the composition.

9. The composition according to anyone of claims 1 to 8, wherein the composition comprises g) in an amount of from about 6.5wt.% to about 12wt.% of the total weight of the composition.

10. The composition according to anyone of claims 1 to 9, wherein the composition comprises k).

11. The composition according to anyone of claims 1 to 10, wherein the composition is in a solid oral dosage form selected from the group consisting of: a powder, a tablet and a capsule.

12. The composition according to anyone of claims 1 to 9, wherein the composition consists of:
a) *Momordica Charantia* extract;
b) *Syzygium Cumini* extract;
c) *Swertia Chirayita* extract;
d) *Trigonella Foenum-Graecum extract;*
e) *Gymnema Sylvestre* extract;
f) *Phyllanthus Emblica* extract;
g) *Neopicrorhiza Scrophulariiflora* extract *and*/*or Picrorhiza Kurroa* extract;
h) *Curcuma longa* extract;
i) *Cinnamomum Verum* extract; and
j) *Piper Nigrum* extract.

13. The composition according to anyone of claims 1 to 11, wherein the composition consists of:
a) *Momordica Charantia* extract;
b) *Syzygium Cumini* extract;
c) *Swertia Chirayita* extract;
d) *Trigonella Foenum-Graecum extract;*
e) *Gymnema Sylvestre* extract;
f) *Phyllanthus Emblica* extract;
g) *Neopicrorhiza Scrophulariiflora* extract *and*/*or Picrorhiza Kurroa* extract;
h) *Curcuma longa* extract;
i) *Cinnamomum Verum* extract;
j) *Piper Nigrum* extract; and
k) Fruit mixture extract, wherein the fruit mixture comprises apple, pear and plum.

14. The composition according to anyone of claims 1 to 13, wherein the composition is in a capsule as a solid oral dosage form and wherein a) to j) are present in an amount of about 505mg.

15. The composition according to anyone of claims 1 to 14 for use in the treatment and/or prevention of diabetes.
